(19) Europäisches Patentamt
European Patent Office
Office européen des brevets

(11) **EP 3 803 990 B1**

(12) **FASCICULE DE BREVET EUROPEEN**

(45) Date de publication et mention
de la délivrance du brevet:
**30.03.2022 Bulletin 2022/13**

(21) Numéro de dépôt: **19731153.3**

(22) Date de dépôt: **23.05.2019**

(51) Classification Internationale des Brevets (IPC):
***H01L 35/30*** *(2006.01)*  ***A61N 1/378*** *(2006.01)*

(52) Classification Coopérative des Brevets (CPC):
**A61N 1/3785; H01L 35/30;** A61N 1/365

(86) Numéro de dépôt international:
**PCT/EP2019/063381**

(87) Numéro de publication internationale:
**WO 2019/224325 (28.11.2019 Gazette 2019/48)**

(54) **GÉNÉRATEUR THERMOÉLECTRIQUE, DISPOSITIF IMPLANTABLE ET PROCÉDÉ ASSOCIÉS**

THERMOELEKTRISCHER GENERATOR, ZUGEHÖRIGE IMPLANTIERBARE VORRICHTUNG UND VERFAHREN

THERMO-ELECTRIC GENERATOR, ASSOCIATED IMPLANTABLE DEVICE AND METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.05.2018 FR 1854398**

(43) Date de publication de la demande:
**14.04.2021 Bulletin 2021/15**

(73) Titulaires:
• **Université Grenoble Alpes**
**38400 Saint-Martin-d'Hères (FR)**
• **INSTITUT NATIONAL DE LA SANTE ET DE LA RECHERCHE MEDICALE - INSERM**
**75013 Paris (FR)**
• **Centre Hospitalier Universitaire Grenoble Alpes**
**38700 La Tronche (FR)**

(72) Inventeurs:
• **ZEBDA, Abdelkader**
**38100 GRENOBLE (FR)**
• **CINQUIN, Philippe**
**38330 SAINT NAZAIRE LES EYMES (FR)**
• **ALCARAZ, Jean-Pierre**
**38530 PONTCHARRA (FR)**
• **MARTIN, Donald**
**38610 GIERES (FR)**

• **BAKRI, Aziz**
**38100 GRENOBLE (FR)**
• **KHALEF, Nawel**
**38400 Saint Martin d'Hères (FR)**

(74) Mandataire: **Cabinet Chaillot**
**16/20, avenue de l'Agent Sarre**
**B.P. 74**
**92703 Colombes Cedex (FR)**

(56) Documents cités:
EP-A2- 2 124 267          WO-A1-2006/017226
DE-A1-102006 027 350      US-A- 6 131 581
US-A1- 2005 038 483       US-A1- 2015 054 468

• MUEHLBAUER M J ET AL: "Thermoelectric enzyme sensor for measuring blood glucose", BIOSENSORS AND BIOELECTRONICS, ELSEVIER SCIENCE LTD. UK, AMSTERDAM, NL, vol. 5, no. 1, 1 janvier 1990 (1990-01-01) , pages 1-12, XP026594272, ISSN: 0956-5663, DOI: 10.1016/0956-5663(90)80022-6 [extrait le 1990-01-01]
• ACHRAF AMAR ET AL: "Power Approaches for Implantable Medical Devices", SENSORS, vol. 15, no. 11, 13 novembre 2015 (2015-11-13), pages 28889-28914, XP055544879, CH ISSN: 1424-8220, DOI: 10.3390/s151128889

**Description**

La présente invention concerne un générateur thermoélectrique. La présente invention concerne également un procédé de génération d'une différence de potentiel associé.

Des dispositifs sont utilisés dans un grand nombre d'applications pour générer un courant électrique ou une tension électrique pour alimenter des dispositifs spécifiques ou pour récupérer une énergie présente dans l'environnement dans lequel ils sont placés. Cependant, ces dispositifs sont en général adaptés à certaines applications particulières et inadaptés à d'autres.

Par exemple, des biopiles catalysant des réactions d'oxydoréduction entre des espèces chimiques du corps humain ont été proposées pour alimenter des dispositifs implantés tels que des stimulateurs cardiaques. Toutefois, ces biopiles ne sont adaptées qu'à être utilisées dans des environnements dans lesquels des oxydants et des réducteurs susceptibles de réagir ensemble sont disponibles.

Dans d'autres domaines, des générateurs thermoélectriques sont utilisés pour générer un courant électrique à partir de différences de températures entre deux zones. Par exemple, de tels générateurs permettent de récupérer une partie de l'énergie thermique issue de certains procédés en la convertissant partiellement en énergie électrique.

Les générateurs thermoélectriques comprennent en général deux électrodes présentant chacune une première extrémité et une deuxième extrémité. Chaque électrode est réalisée en un matériau semi-conducteur dopé, le type de dopage étant différent d'une électrode à l'autre. Les premières extrémités sont connectées électriquement l'une à l'autre. Lorsqu'une différence de température est imposée entre la première extrémité et la deuxième extrémité de chaque électrode, une différence de potentiel électrique apparaît entre les deux électrodes, qui permet donc la génération d'un courant électrique. Ainsi, lorsque l'une des extrémités est en contact avec un objet chaud, tel qu'une sortie d'échappement d'une installation industrielle, et l'autre extrémité de chaque électrode en contact avec un objet froid, tel qu'un mur extérieur de la même installation ou une étendue d'eau, une partie de l'énergie thermique présente dans l'échappement est récupérée.

Toutefois, la puissance électrique délivrée par les générateurs thermoélectriques dépend de la différence de température entre les premières et deuxièmes extrémités. Plus cette différence de température est faible, plus la puissance électrique délivrée est faible. Les générateurs thermoélectriques sont donc peu adaptés à des applications dans lesquelles les différences de température disponibles sont faibles.

MUEHLBAUER M J ET AL, "Thermoelectric enzyme sensor for measuring blood glucose", BIOSENSORS AND BIOELECTRONICS, ELSEVIER SCIENCE LTD. UK, AMSTERDAM, NL, vol. 5, no. 1, doi:10.1016/0956-5663(90)80022-6, ISSN 0956-5663, (19900101), pages 1 - 12, (19900101), divulgue un générateur thermoélectrique selon l'état de la technique.

Il existe donc un besoin pour un générateur électrique qui soit adapté à un plus grand nombre d'applications que les générateurs de l'état de la technique.

A cet effet, il est proposé un générateur thermoélectrique destiné à être plongé dans un fluide contenant au moins une première espèce chimique, le générateur thermoélectrique comprenant au moins deux électrodes, chaque électrode présentant une première extrémité et une deuxième extrémité, les premières extrémités étant électriquement connectées l'une à l'autre, le générateur étant configuré pour générer une différence de potentiel électrique entre les deux deuxièmes extrémités lorsqu'une différence de température est imposée entre chaque première extrémité et la deuxième extrémité de la même électrode, la différence de température étant telle qu'une extrémité, appelée « extrémité chaude », parmi la première extrémité et la deuxième extrémité de chaque électrode présente une température strictement supérieure à la température de l'autre extrémité, appelée « extrémité froide », parmi la première extrémité et la deuxième extrémité de la même électrode. L'extrémité chaude d'au moins une électrode comporte au moins un élément choisi parmi l'ensemble formé d'un micro-organisme et d'une enzyme, l'élément étant propre à provoquer au moins une réaction exothermique mettant en jeu la première espèce chimique,
le générateur thermoélectrique étant caractérisé en ce qu'il comprend en outre un bloc en contact avec l'extrémité chaude d'au moins une électrode, le bloc comprenant une matrice, la matrice étant notamment réalisée en un matériau polymère, l'élément étant en forme de particules encapsulées dans la matrice.

Selon des modes de réalisation particuliers, le générateur thermoélectrique comprend une ou plusieurs des caractéristiques suivantes, prise(s) isolément ou selon toutes les combinaisons techniquement possibles :

- le générateur comprend un boîtier délimitant une chambre accueillant les deux extrémités chaudes, la chambre accueillant en outre l'élément, le boîtier étant configuré pour être traversé au moins par la première espèce chimique.
- le boîtier est réalisé en un matériau poreux.
- le générateur comprend une pompe propre à injecter un flux de fluide depuis l'extérieur du boîtier jusque dans la chambre.
- le boîtier est réalisé en un matériau polymère.
- le matériaux poreux est constitué d'une matrice 3D poreuse.

- la matrice 3D poreuse est fabriquée par impression 3D à partir d'une pâte visqueuse de poudre de particules métalliques et d'un liant visqueux.
- la taille des pores de la matrice 3D varie de 50 μm jusqu'à 1mm.
- les particules de poudre de particules métalliques sont : des particules de carbone, des particules de nanotubes de carbone, des particules de charbon actif, des particules de graphène, des particules d'aluminium.
- le liant visqueux est l'un ou un mélange de plusieurs éléments parmi les composés suivants dérivés de cellulose, alginate, agarose, alcool polyvinylique, polychlorure de vinyle.
- la forme géométrique de la matrice est l'une parmi un cube, un cylindre, un pavé.
- une face de la matrice est fermée par un isolant thermique, les autres faces de la matrice étant fermées par un matériau thermoconducteur.
- le matériau thermoconducteur est par exemple l'aluminium.
- l'isolant thermique est par exemple le pyralène.
- la matrice possède une entrée de fluide et une sortie de fluide.
- la matrice 3D comprend des micro-organismes qui forment un biofilm à l'intérieur de la matrice 3D.
- les micro-organismes sont mélangés avec une pâte visqueuse de poudre de particules métalliques et un liant visqueux.
- le composite formé par les micro-organismes mélangés avec une pâte visqueuse de poudre de particules métalliques et un liant visqueux est utilisé pour fabriquer une matrice 3D poreuse fabriquée par impression 3D.
- 70% à 80% de la surface de la matrice 3D est couverte d'une couche thermiquement isolante.
- la couche thermiquement isolante est par exemple le parylène.
- la matrice poreuse est un bloc métal, par exemple aluminium, carbone, titane, or ou équivalents, le bloc métal étant fabriqué par impression 3D.
- l'élément est en forme de particules compactées formant un solide poreux.
- l'élément est propre à provoquer une première réaction exothermique et une deuxième réaction exothermique, la première réaction générant au moins un produit par réaction de la première espèce chimique, la deuxième réaction étant une réaction mettant en jeu le produit de la première réaction.
- au moins un élément est un micro-organisme, au moins une des propriétés suivantes étant vérifiée :

  • le micro-organisme comprend une levure, et
  • le micro-organisme comprend une bactérie.

- la première espèce chimique est choisie parmi l'ensemble formé de : l'urée, un alcool et un sucre.
- au moins une espèce est le glucose.

L'utilisation d'une matrice poreuse 3D permet avantageusement d'augmenter le nombre ou la densité des micro-organismes par la création de biofilms bactériens ou de micro-organismes à la surface et à l'intérieur de la matrice poreuse 3D.
La densité de micro-organismes est en effet plus importante dans un biofilm que dans le cas où les micro-organismes sont en solution.
La porosité de la matrice 3D permet également avantageusement l'alimentation en eau et substrats des micro-organismes.
Le matériau constituant la matrice poreuse 3D a de préférence une chaleur spécifique faible et une conduction thermique élevée, ce qui est par exemple le cas des métaux et de leurs composites, afin de rendre les pertes thermiques minimales dans le générateur thermoélectrique de l'invention, pour optimiser la chaleur dégagée par les microorganismes.
Il est également proposé un dispositif implantable configuré pour être implanté dans le corps humain ou animal, comprenant un générateur thermoélectrique tel que précédemment défini, dans lequel le fluide est un fluide corporel du corps humain, la première espèce chimique étant notamment le glucose.
Selon des modes de réalisation particuliers, le dispositif implantable comprend une ou plusieurs des caractéristiques suivantes, prise(s) isolément ou selon toutes les combinaisons techniquement possibles :

- au moins un élément comprend une enzyme, l'élément comprenant une glucose oxydase et une catalase.
- le dispositif implantable est un stimulateur cardiaque.

Il est également proposé un procédé de génération d'une différence de potentiel électrique, comprenant des étapes de :

• fourniture d'un générateur thermoélectrique comprenant au moins deux électrodes, chaque électrode présentant une première extrémité et une deuxième extrémité, les premières extrémités étant électriquement connectées l'une à l'autre, le générateur étant configuré pour générer une différence de potentiel électrique entre les deux deuxièmes extrémités lorsqu'une différence de température est imposée entre chaque première extrémité et la deuxième extrémité de la même électrode, la différence de température étant telle qu'une extrémité, appelée « extrémité chaude », parmi la première extrémité et la deuxième extrémité de chaque électrode présente une température strictement supérieure à la température de l'autre extrémité, appelée « extrémité froide » parmi la première extrémité et la deuxième extrémité de la même électrode, l'extrémité chaude d'au moins une électrode comportant au moins un élément choisi parmi l'ensemble formé d'un micro-organisme et d'une enzyme, le générateur thermoélectrique comprenant en outre un bloc en contact avec l'extrémité chaude d'au moins une électrode, le bloc comprenant une matrice, la matrice étant notamment réalisée en un matériau polymère, l'élément étant en forme de particules encapsulées dans la matrice,

- trempage du générateur thermoélectrique dans un fluide comprenant au moins une première espèce chimique,
- mise en œuvre, par l'élément, d'au moins une réaction exothermique mettant en jeu la première espèce chimique,
- apparition d'une différence de température entre l'extrémité chaude et l'extrémité froide de l'électrode comportant l'élément, et
- apparition d'une différence de potentiel électrique entre les deux deuxièmes extrémités.

Des caractéristiques et avantages de l'invention apparaîtront clairement à la lecture de la description qui va suivre, donnée uniquement à titre d'exemple non limitatif et faite en référence aux dessins annexés, sur lesquels :

- la figure 1 est une représentation schématique d'un exemple de dispositif implantable comprenant un générateur thermoélectrique selon l'invention,
- la figure 2 est un ordinogramme d'un exemple de procédé de génération d'une différence de potentiel électrique mis en œuvre à l'aide du générateur thermoélectrique de la figure 1,
- la figure 3 représente une matrice poreuse 3D d'un générateur thermoélectrique selon un mode de réalisation de la présente invention,
- la figure 4 représente un générateur thermoélectrique selon un mode de réalisation de la présente invention.

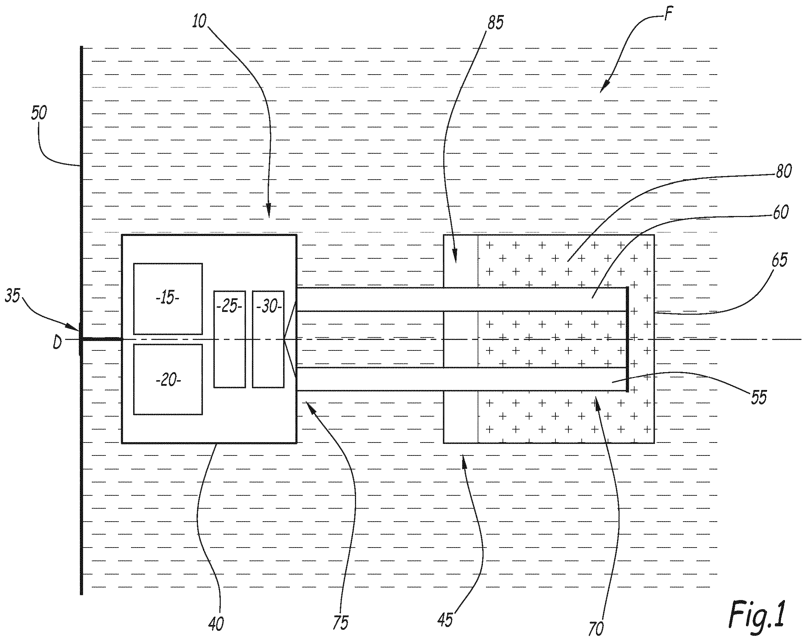

Un dispositif implantable 10 est représenté sur la figure 1.

Il est entendu par dispositif implantable 10 que le dispositif implantable 10 est prévu pour être implanté dans le corps d'un animal, par exemple d'un être humain. En particulier, il est entendu par « implantable » que le dispositif implantable 10 est prévu pour séjourner dans le corps pendant une durée strictement supérieure à une semaine, de préférence strictement supérieure à un mois, de préférence supérieure ou égale à un an.

Le dispositif implantable 10 est, par exemple, configuré pour être placé dans une cavité corporelle du corps contenant un fluide F. L'intestin ou l'estomac sont des exemples de cavités corporelles.

En variante, le dispositif implantable 10 est configuré pour être implanté hors d'une cavité corporelle, par exemple dans un muscle ou sous la peau du patient.

Le dispositif implantable 10 est configuré pour stimuler un organe du corps. Par exemple, l'organe est le cœur et le dispositif implantable 10 est alors un stimulateur cardiaque.

Il est à noter que des dispositifs implantables 10 remplissant d'autres fonctions que la stimulation sont également envisageables. Par exemple, un dispositif implantable 10 comprenant un capteur propre à mesurer des valeurs d'un paramètre physiologique du corps tel qu'un taux d'un marqueur spécifique dans le fluide corporel F et à transmettre les valeurs mesurées à un autre dispositif est envisageable.

Il est également envisageable que le dispositif implantable 10 comprenne une réserve d'une substance active telle qu'un médicament et soit prévu pour injecter la substance active dans le corps.

De manière générale, le dispositif implantable 10 est susceptible de remplir toute fonction requérant une alimentation électrique du dispositif implantable 10.

Le dispositif implantable 10 comporte, par exemple, un dispositif de stimulation 15, un contrôleur 20, un accumulateur 25, un convertisseur 30, une ancre 35, un premier boîtier 40 et un générateur thermoélectrique 45.

Le fluide F est un fluide corporel remplissant partiellement ou totalement la cavité corporelle.

Le fluide F est, par exemple, un liquide.

Par exemple, le fluide F est le sang. En variante, le fluide F est le liquide péritonéal. Selon une autre variante, le fluide F est le liquide contenu dans l'estomac, notamment un mélange de sucs gastriques.

Le liquide extracellulaire ou le fluide contenu dans l'intestin du patient sont d'autres exemples de fluide F.

Le fluide F contient au moins une première espèce chimique C1. Selon un mode de réalisation particulier, le fluide F contient la première espèce chimique C1 et au moins une deuxième espèce chimique C2.

La première espèce chimique C1 est une espèce chimique naturellement présente dans le fluide F.

La première espèce chimique C1 est, par exemple, le glucose.

Il est à noter qu'un grand nombre d'espèces chimiques sont susceptibles de jouer le rôle de première espèce chimique C1. Bien entendu, entrent en ligne de compte les espèces chimiques susceptibles d'être présentes dans le fluide au contact ou dans l'environnement du dispositif. Par exemple, la première espèce C1 est susceptible d'être choisie parmi l'ensemble formé du pyruvate, de l'acide ascorbique, de l'urée, d'un alcool et d'un sucre. Le glucose a été mentionné, on peut ajouter les autres monosaccharides que sont le fructose, le galactose et le mannose (issus de la digestion des glucides ou sucres, et/ou apportés par l'alimentation), ou encore le glycogène. Selon un autre mode de réalisation particulier, le sucre est le saccharose (notamment si le dispositif est dans l'estomac).

Lorsque le fluide F est le liquide extra-cellulaire, par exemple lorsque le dispositif implantable 10 est destiné à être implanté sous la peau du patient, la première espèce chimique C1 est, par exemple, le glucose, ou encore le pyruvate.

La deuxième espèce chimique C2 est une espèce chimique naturellement présente dans le fluide F.

La deuxième espèce chimique C2 est, par exemple, le dioxygène.

Il est à noter que les première et deuxième espèces chimiques C1 et C2 sont susceptibles d'être choisies parmi toutes les espèces naturellement présentes dans le fluide F. Selon un mode de réalisation, au moins une parmi la première espèce chimique C1 et la deuxième espèce chimique C2 est une molécule organique provenant de l'alimentation et/ou issue de la digestion ou transformation d'aliments, et présente dans l'estomac et/ou l'intestin du patient.

Dans certaines applications, la première et/ou la deuxième espèce chimique C1, C2 sont susceptibles d'être des espèces naturellement présente dans une biomasse, par exemple des espèces libérées lors de la décomposition de la biomasse.

Le dispositif de stimulation 15 est configuré pour stimuler l'organe.

Par exemple, le dispositif de simulation 15 est configuré pour stimuler électriquement l'organe. En particulier, le dispositif de stimulation 15 comporte une électrode propre à être électriquement connectée à l'organe et est configuré pour imposer une tension électrique entre l'électrode et le premier boîtier 40.

En variante, le dispositif de stimulation 15 est configuré pour stimuler mécaniquement l'organe.

Le contrôleur 20 est configuré pour commander la stimulation de l'organe par le dispositif de stimulation 15. Par exemple, le contrôleur 20 est propre à calculer une fréquence de stimulation, ou à détecter un phénomène physiologique du corps tel qu'une anomalie du rythme cardiaque et pour commander la stimulation lorsque l'anomalie est détectée.

Le contrôleur 20 est, par exemple, une unité de traitement de données comprenant une mémoire et un processeur.

Il est à noter que d'autres types de contrôleur 20 sont susceptibles d'être utilisés. Par exemple, selon une variante, le contrôleur 20 est un composant logique programmable. Un composant logique programmable, également appelé circuit logique programmable ou réseau logique programmable, est un circuit intégré logique qui peut être reprogrammé après sa fabrication. Selon une autre variante, le contrôleur 20 est un ASIC. Un ASIC (de l'Anglais «Application-Specific Integrated Circuit », signifiant littéralement « circuit intégré propre à une application ») est un circuit intégré spécialisé et dédié à une application spécifique.

L'accumulateur 25 est configuré pour stocker de l'énergie électrique. L'accumulateur 25 est, par exemple, une supercapacité, ou encore un ensemble de batteries.

Le convertisseur 30 est configuré pour recevoir du générateur thermoélectrique 45 une première tension électrique et pour générer en réponse une deuxième tension électrique d'alimentation de l'accumulateur 25, du contrôleur 20 et du dispositif de stimulation 15.

L'ancre 35 est configurée pour maintenir le dispositif implantable 10 en position lorsque le dispositif implantable 10 est implanté dans le corps. Par exemple, l'ancre 35 est configurée pour être fixée à une paroi 50 du corps telle qu'une paroi stomacale.

Il est à noter que de nombreux types d'ancres 35 sont susceptibles d'être utilisées.

De nombreux emplacements sont susceptibles d'être envisagés pour fixer l'ancre 35 et le dispositif implantable 10.

Le premier boîtier 40 est configuré pour isoler le dispositif de stimulation 15, le contrôleur 20, l'accumulateur 25 et le convertisseur 30 de l'extérieur du premier boîtier 40, notamment du fluide F.

Le générateur thermoélectrique 45 est prévu pour être plongé dans le fluide F. En particulier, le générateur thermoélectrique 45 est prévu pour être au moins partiellement en contact avec le fluide F.

Le générateur thermoélectrique 45 est configuré pour générer la première différence de potentiel électrique. Par exemple, le générateur thermoélectrique 45 est configuré pour générer la première différence de potentiel électrique et pour alimenter le convertisseur 30 avec la première différence de potentiel électrique.

Le générateur thermoélectrique 45 comporte une première électrode 55, une deuxième électrode 60 et un deuxième boîtier 65.

Chaque électrode 55, 60 présente une première extrémité 70 et une deuxième extrémité 75.

Par exemple, chaque électrode 55, 60 s'étend selon une direction principale D entre la première extrémité 70 et la deuxième extrémité 75.

Selon un mode de réalisation, chaque électrode 55, 60 présente une section de forme rectangulaire dans un plan perpendiculaire à la direction principale D. Il est à noter que la forme de la section est susceptible de varier d'un mode de réalisation à un autre.

Chaque électrode 55, 60 est réalisée en un matériau semiconducteur.

Il est entendu par «matériau semi-conducteur» un matériau présentant une valeur de gap strictement supérieure à zéro et inférieure ou égale à 6,5 eV.

Il est entendu par « valeur de gap » d'un matériau la valeur de la largeur de la bande interdite entre la bande de valence et la bande de conduction dans le matériau. La valeur de gap d'un matériau est, par exemple, exprimée en électron-volts (eV).

La bande de valence est définie comme étant, parmi les bandes d'énergie permises pour un électron le matériau considéré, la bande qui présente l'énergie la plus haute tout en étant remplie complètement à une température inférieure ou égale à 20 K.

Un premier niveau d'énergie est défini pour chaque bande de valence. Le premier niveau d'énergie est le niveau d'énergie le plus élevé de la bande de valence.

La bande de conduction est définie comme étant, parmi les bandes d'énergie permises pour un électron dans le matériau, la bande qui présente l'énergie la plus basse tout en n'étant pas remplie à une température inférieure ou égale à 20 K.

Un deuxième niveau d'énergie est défini pour chaque bande de conduction. Le deuxième niveau d'énergie est le niveau d'énergie le plus bas de la bande de conduction.

Ainsi, chaque valeur de gap est mesurée entre le premier niveau d'énergie et le deuxième niveau d'énergie du matériau considéré.

Chaque électrode 55, 60 est, par exemple, réalisée en un unique matériau semiconducteur.

Selon un mode de réalisation, au moins une électrode 55, 60 comporte une pluralité de portions, chaque portion étant réalisée en un matériau semiconducteur différents des autres portions de l'électrode 55, 60 considérée.

Chaque matériau semiconducteur est, par exemple, choisi parmi le groupe formé de : un alliage de tellure et d'un autre élément, et un polymère thermoélectrique.

Lorsque le matériau semiconducteur est un alliage de tellure et d'un autre élément, l'autre élément est, par exemple, le bismuth ou l'antimoine.

Il est à noter que d'autres matériaux semiconducteurs sont susceptibles d'être utilisés.

La polyaniline et la polypyrrole sont des exemples de polymères thermoélectriques, cependant d'autres polymères thermoélectriques sont susceptibles d'être envisagés.

Les portions sont électriquement connectées entre elles. En particulier, les portions sont connectées en série entre la première extrémité 70 et la deuxième extrémité 75. Par exemple, les portions d'une même électrode 55, 60 sont alignées selon la direction principale D.

Chaque deuxième extrémité 75 est, par exemple, connectée électriquement au convertisseur 30.

Il est défini, pour chaque électrode 55, 60, une extrémité chaude et une extrémité froide.

Chacune de l'extrémité chaude et de l'extrémité froide est choisie parmi la première extrémité 70 et la deuxième extrémité 75.

L'extrémité chaude et l'extrémité froide sont définies par le fait que le générateur thermoélectrique 45 est configuré pour générer la première différence de potentiel électrique lorsqu'une différence de température est imposée entre l'extrémité chaude et l'extrémité froide de chaque électrode 55, 60, la température de l'extrémité chaude étant strictement supérieure à la température de l'extrémité froide.

Dans l'exemple représenté sur la figure 1, l'extrémité chaude de chaque électrode 55, 60 est la première extrémité 70 et l'extrémité froide de chaque électrode 55, 60 est la deuxième extrémité 75.

Il est à noter que des modes de réalisation dans lesquels l'extrémité froide de chaque électrode 55, 60 est la première extrémité 70 et l'extrémité chaude de chaque électrode 55, 60 est la deuxième extrémité 75 sont également envisageables.

La première extrémité 70 de chaque électrode 55, 60 est électriquement connectée à la première extrémité 70 de l'autre électrode 55, 60.

Les premières extrémités 70 sont, par exemple, électriquement connectées l'une à l'autre par un conducteur métallique, par exemple un conducteur réalisé en or, en cuivre, ou dans un autre matériau métallique.

La première électrode 55 présente un premier type de dopage. Par exemple, chaque portion de la première électrode présente le premier type de dopage.

Le dopage est défini comme étant la présence, dans un matériau, d'impuretés apportant des porteurs de charge libres. Les impuretés sont, par exemple, des atomes d'un élément qui n'est pas présent naturellement dans le matériau.

Lorsque la présence des impuretés augmente la densité volumique de trous présents dans le matériau par rapport au matériau non dopé, le dopage est de type p.

Lorsque la présence des impuretés augmente la densité volumique d'électrons libres présents dans le matériau par rapport au matériau non dopé, le dopage est de type n.

Le premier type de dopage est choisi parmi le dopage de type n et le dopage de type p. Par exemple, le premier type de dopage est le dopage de type n.

La deuxième électrode 60 présente un dopage d'un deuxième type choisi parmi le dopage de type p et le dopage de type n. En particulier, le deuxième type de dopage est différent du premier type de dopage. Par exemple, le deuxième type de dopage est le dopage de type p.

L'extrémité chaude d'au moins une électrode 55, 60 comporte un élément 80. Par exemple, l'extrémité chaude de chaque électrode 55, 60 comporte un élément 80.

A contrario, l'extrémité froide de chaque électrode 55, 60 est dépourvue d'élément 80.

Selon un mode de réalisation, le générateur thermoélectrique 45 comporte un unique élément 80 commun à toutes les électrodes 55, 60.

L'élément 80 est configuré pour générer une différence de température entre la première extrémité 70 de l'électrode 55, 60 correspondante et la deuxième extrémité 75 de la même électrode 55, 60. En particulier, l'élément 80 est configuré pour générer une différence de température telle que la température de l'extrémité chaude est strictement supérieure à la température de l'extrémité froide.

En particulier, l'élément 80 est propre à provoquer au moins une réaction exothermique mettant en jeu la première espèce chimique C1.

Selon un mode de réalisation, l'élément 80 est propre à provoquer au moins une première réaction exothermique et au moins une deuxième réaction exothermique.

Il est à noter que le nombre de réactions exothermiques provoquées par l'élément 80 est susceptible de varier. Par exemple, des modes de réalisation dans lesquels l'élément 80 est propre à provoquer une unique première réaction exothermique sont envisageables, de même que des modes de réalisation dans lesquels l'élément 80 est propre à provoquer trois réactions exothermiques ou plus.

Selon un mode de réalisation, chaque élément 80 est un catalyseur de la ou les réactions exothermiques que l'élément 80 est propre à générer. Un catalyseur est une substance qui provoque une réaction chimique ou augmente la vitesse d'une réaction chimique sans être consommée par cette réaction chimique.

Chaque élément 80 est choisi parmi l'ensemble formé d'un micro-organisme et d'une enzyme. Par exemple, l'élément 80 est une enzyme, un mélange de plusieurs enzymes, un micro-organisme, un mélange de plusieurs micro-organismes ou un mélange d'au moins une enzyme et au moins un micro-organisme.

Une enzyme est une protéine dotée de propriétés catalytiques.

Selon un mode de réalisation, l'élément 80 est une enzyme. Par exemple, l'élément 80 est un mélange de deux enzymes différentes. En particulier, l'élément 80 comprend une glucose oxydase et une catalase.

La glucose oxydase (également dénommée par les acronymes GOx et GOD) est une enzyme oxydo-réductase, de nomenclature EC EC 1.1.3.4, qui catalyse l'oxydation du glucose en peroxyde d'hydrogène et en acide gluconique.

La nomenclature EC (EC est le sigle de Enzyme Commission, la Commission des enzymes) est une classification numérique des enzymes, basée sur la réaction chimique qu'elles catalysent.

Une catalase (du grec kataluein, « dissoudre ») est une oxydoréductase héminique qui catalyse la dismutation du peroxyde d'hydrogène en eau et dioxygène.

La dismutation est une réaction de parallélisme dans laquelle une espèce chimique joue à la fois le rôle d'oxydant et de réducteur. La dismutation du peroxyde d'hydrogène en eau et dioxygène est définie par l'équation-bilan :

$$2H_2O_2 \rightarrow O_2 + 2H_2O \qquad \text{(Equation 1)}$$

Selon une variante, l'enzyme comprend au moins une enzyme choisie parmi une saccarase, une fructose oxydase et une galactose oxydase. Par exemple, l'enzyme comprend une glucose oxydase, une catalase, une fructose oxydase, une saccarase et une galactose oxydase.

Il est entendu par « micro-organisme » un ensemble d'organismes vivants, chaque organisme ayant une dimension maximale inférieure ou égale à 100 microns, par exemple inférieure ou égale à 50 microns, notamment comprise entre 5 microns et 20 microns.

Chaque micro-organisme est propre à provoquer une réaction exothermique mettant en jeu la première espèce chimique C1.

Certains micro-organismes sont, par exemple, propres à provoquer une réaction exothermique d'oxydation de la première espèce chimique C1. Il est à noter que des réactions exothermiques qui ne sont pas des réactions d'oxydoréduction sont également susceptibles d'être envisagées.

Le micro-organisme comprend, par exemple, au moins une levure. Par exemple, le micro-organisme comprend un mélange de plusieurs levures différentes.

Une levure est un champignon unicellulaire.

Selon un mode de réalisation spécifique, la levure est, par exemple, Saccharomyces cerevisiae. Il est à noter que d'autres types de levures sont également envisageables.

En variante, le micro-organisme comprend au moins une bactérie. Par exemple, le micro-organisme comprend un mélange de plusieurs bactéries différentes.

De nombreux types de bactéries sont susceptibles d'être envisagées pour le micro-organisme.

Par exemple, le micro-organisme comprend un type de bactérie commensale ou un ensemble (mélange) de plusieurs types de bactéries commensales. Une bactérie commensale est une bactérie qui colonise l'organisme (généralement la peau ou les muqueuses) d'un hôte tel qu'un être humain, sans provoquer de maladie. La bactérie *Escherichia coli* est un exemple de bactérie commensale utilisable dans l'invention.

Lorsque le dispositif implantable 10 est prévu pour être implanté dans l'intestin du patient, le micro-organisme comprend par exemple au moins un type de bactéries qui sont naturellement présentes dans l'intestin.

Il est à noter que d'autres types de bactéries sont également envisageables.

La première réaction exothermique génère au moins un produit P par réaction de la première espèce chimique C1.

Par exemple, la première réaction exothermique est une réaction entre la première espèce chimique C1 et une deuxième espèce chimique C2. Une réaction d'oxydation est un exemple de réaction entre la première espèce chimique C1 et la deuxième espèce chimique C2. Toutefois, des réactions exothermiques qui ne sont pas des réactions d'oxydoréduction sont également susceptibles d'être envisagées.

Selon un mode de mise en œuvre, la première réaction exothermique est une réaction d'hydrolyse de la première espèce chimique ou de la deuxième espèce chimique. Selon un autre mode de mise en œuvre, la première réaction exothermique est une réaction de dismutation de la première espèce chimique ou de la deuxième espèce chimique.

Lorsque la première espèce chimique C1 est le glucose, la première réaction est, par exemple, une décomposition du glucose, en présence de dioxygène, en un mélange d'acide gluconique et de peroxyde d'hydrogène $H_2O_2$. Dans ce cas, la première réaction exothermique génère deux produits P, qui sont l'acide gluconique et le peroxyde d'hydrogène.

Lorsque l'élément 80 comprend un ensemble de bactéries, la première réaction exothermique est, par exemple, une réaction métabolique des bactéries, c'est-à-dire une réaction mise en œuvre par les bactéries afin de se maintenir en vie, de se développer, de se reproduire, ou encore de répondre aux stimuli de l'environnement auxquels les bactéries sont exposées.

Il est à noter qu'un grand nombre de premières réactions exothermiques sont envisageables.

La deuxième réaction exothermique est une réaction exothermique mettant en jeu au moins un produit P généré par la première réaction. Par exemple, la deuxième réaction exothermique est une réaction entre le produit P et une deuxième espèce chimique C2.

Selon un mode de réalisation, la deuxième réaction exothermique est une réaction mettant en jeu uniquement le produit P.

Par exemple, la deuxième réaction exothermique est une réaction de décomposition du produit P. Un exemple de réaction de décomposition est la décomposition du peroxyde d'hydrogène en un mélange d'eau et de dihydrogène.

L'élément 80 est, par exemple, en contact avec l'extrémité chaude d'au moins une électrode 55, 60.

Dans le mode de réalisation représenté sur la figure 1, l'élément 80 entoure l'extrémité chaude de l'électrode 55, 60 correspondante. Par exemple, l'élément 80 entoure l'extrémité chaude, en l'occurrence la première extrémité 70, de chacune des deux électrodes 55, 60.

En particulier, l'élément 80 entoure l'extrémité chaude de l'électrode 55, 60 correspondante dans un plan perpendiculaire à la direction principale D. L'élément 80 est, en outre, interposé entre le deuxième boîtier 65 et l'extrémité chaude de telle sorte que l'extrémité chaude, l'élément 80 et le deuxième boîtier 65 sont alignés dans cet ordre selon la direction principale D.

L'élément 80 est, par exemple, un solide poreux.

L'élément 80 est, notamment, configuré pour être traversé par des molécules présentes dans le fluide F. En particulier, l'élément 80 est prévu pour que les pores permettent le passage de la première et/ou de la deuxième espèce chimique C1, C2.

Un solide poreux présentant des pores dont la dimension minimale est supérieure ou égale à 1 nanomètre (nm) est un exemple de solide poreux susceptible d'être utilisé comme élément 80.

L'élément 80 est, par exemple, réalisé en un matériau thermiquement isolant.

L'élément 80 est, notamment, réalisé en un matériau biocompatible. La biocompatibilité est la capacité des matériaux à ne pas interférer, ne pas dégrader, le milieu biologique dans lequel ils sont utilisés. Les matériaux biocompatibles sont également appelés biomatériaux.

L'élément 80 présente une épaisseur, mesurée selon une direction perpendiculaire à la surface de l'extrémité chaude, comprise entre 1 micromètre et 2 centimètres.

L'élément 80 est, par exemple, un film recouvrant au moins partiellement la surface de l'extrémité chaude correspondante.

En variante, l'élément 80 est un bloc en contact avec l'extrémité chaude. L'élément 80 est, par exemple, un ensemble de particules.

Selon un mode de réalisation, l'élément 80 est un solide formé par compaction des particules. Le générateur thermoélectrique 45 comporte une matrice, les particules étant encapsulées dans la matrice.

Il est entendu par « matrice » un liant assurant la cohésion entre les différentes particules. En particulier, une masse de matière entourant chaque particule est un exemple de matrice. Par exemple, chaque particule est incluse dans la matrice et solidaire de la matrice.

La matrice est, par exemple, une matrice réalisée en un matériau polymère.

Selon un mode de réalisation, l'élément 80 est intégré dans au moins un matériau semiconducteur composant l'extrémité chaude de l'électrode 55, 60 correspondante. Par exemple, le matériau semiconducteur est poreux et forme une matrice pour les particules qui composent l'élément 80.

L'extrémité chaude d'une électrode 55, 60 dans laquelle le matériau semiconducteur est poreux et forme une matrice pour les particules est, par exemple, réalisée par compaction mécanique de particules, notamment de nanoparticules, semiconductrices et de particules de l'élément 80. Selon un mode de réalisation, l'extrémité chaude est réalisée par compaction mécanique des particules semiconductrices, des particules de l'élément 80 et de polymère.

Selon un autre mode de réalisation envisageable, l'extrémité chaude est réalisée à partir d'une encre comprenant des particules semiconductrices, des particules d'élément 80 et de polymère.

Le deuxième boîtier 65 est configuré pour isoler thermiquement au moins une portion de l'extrémité chaude de chaque électrode 55, 60 du fluide F dans lequel le générateur thermoélectrique 45 baigne.

En particulier, le deuxième boîtier 65 délimite une chambre 85 contenant au moins l'extrémité chaude de chaque électrode 55, 60.

L'élément 80 est, en particulier, accueilli dans la chambre 85.

Dans l'exemple représenté sur la figure 1, l'extrémité froide de chaque électrode 55, 60 n'est pas accueillie dans la chambre 85. En particulier, l'extrémité froide de chaque électrode 55, 60 baigne dans le fluide F.

Il est à noter que des modes de réalisation dans lesquels chaque électrode 55, 60 est entièrement accueillie dans la chambre 85 sont envisageables. Par exemple, le deuxième boîtier 65 comporte une paroi interne divisant la chambre 85 en deux sous-chambres, l'extrémité chaude de chaque électrode 55, 60 étant accueillie dans une sous-chambre et l'extrémité froide de chaque électrode 55, 60 étant accueillie dans l'autre sous-chambre. La paroi interne est alors configurée pour isoler thermiquement les deux sous-chambres l'une de l'autre.

Selon un autre mode de réalisation, la chambre 85 est délimitée par le deuxième boîtier 65 et par une face d'une électrode 55, 60. Dans ce cas, le deuxième boîtier 65 est, par exemple, fixé à l'électrode 55, 60 et configuré pour maintenir l'élément 80 correspondant en contact avec l'électrode 55, 60.

Le deuxième boîtier 65 est configuré pour être traversé par la première espèce chimique C1. Par exemple, le deuxième boîtier 65 comporte une pompe propre à injecter un flux du fluide F dans la chambre 85.

La pompe est, par exemple, une pompe à membrane piézoélectrique.

En variante, le deuxième boîtier 65 est réalisé en un matériau poreux propre à être traversé par le fluide F.

Selon une autre variante, le deuxième boîtier 65 est configuré pour être traversé uniquement par la première espèce chimique C1.

Le deuxième boîtier 65 est, par exemple, réalisé en un matériau polymère, notamment un matériau polymère poreux propre à être traversé par les espèces chimiques C1 et C2.

Dans l'exemple précédent, le générateur thermoélectrique 45 est décrit dans le cas où le générateur thermoélectrique 45 est intégré à un dispositif implantable 10. Il est à noter que des modes de réalisation dans lesquels le générateur thermoélectrique 45 est destiné à être plongé dans un fluide F qui n'est pas un fluide corporel sont également envisageables.

Par exemple, le fluide F est un fluide rejeté par une installation industrielle ou agricole. Dans ce cas, la première espèce chimique C1 est, par exemple, choisie parmi l'ensemble formé de l'urée, d'un sucre, d'un alcool et de la biomasse.

Selon une variante, le fluide F est un flux d'eaux usées telles qu'un tout-à-l'égout, notamment des eaux usées de cuisine ou de sanitaires.

Dans ce cas, la première espèce chimique C1 est, par exemple, choisie parmi l'ensemble formé de l'urée, d'un sucre et d'un alcool.

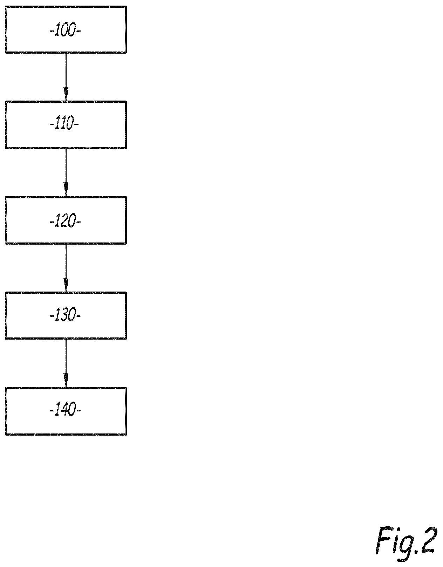

Le fonctionnement du générateur thermoélectrique 45 va maintenant être décrit en référence à la figure 2.

Un ordinogramme des étapes d'un exemple de procédé de génération d'une différence de potentiel est représenté sur la figure 2.

Le procédé de génération comporte une étape 100 de fourniture, une étape 110 de trempage, une étape 120 de mise en œuvre, une étape 130 d'apparition d'une différence de température et une étape 140 d'apparition d'une différence de potentiel électrique.

Lors de l'étape de fourniture 100, un générateur thermoélectrique 45 est fourni.

Lors de l'étape de trempage 110, le générateur thermoélectrique 45 est plongé dans le fluide F.

Par exemple, le dispositif implantable 10 est implanté dans le corps et fixé grâce à l'ancre 35 dans une cavité corporelle de telle manière que le générateur thermoélectrique 45 baigne dans le fluide F qui remplit la cavité corporelle.

Lorsque le fluide F n'est pas un fluide corporel, le générateur thermoélectrique 45 est fixé en position de manière à baigner dans le fluide F, par exemple fixé à une paroi intérieure d'un tuyau destiné à contenir le fluide F.

Lors de l'étape de mise en œuvre 120, au moins la première réaction exothermique est mise en œuvre par l'élément 80. Par exemple, le fluide F vient en contact avec l'élément 80 et la première réaction exothermique est provoquée par l'élément 80.

En particulier, l'élément 80 provoque la première et chaque deuxième réaction exothermique. Plus précisément, l'élément 80 provoque, à partir au moins de la première espèce chimique, la première réaction exothermique, qui entraîne l'apparition du produit P. L'élément 80 provoque, en outre, la deuxième réaction exothermique à partir du produit P généré par la première réaction exothermique.

Il est à noter qu'un grand nombre de réactions exothermiques sont susceptibles d'être utilisées. Par exemple, lorsque l'élément 80 comprend une glucose oxydase et une catalase, une catalase, une fructose oxydase, une saccarase et une galactose oxydase, l'élément 80 provoque l'oxydation du glucose en acide gluconique et en peroxyde d'hydrogène, le peroxyde d'hydrogène étant ensuite décomposé grâce à la catalase.

Lorsque l'élément 80 comprend une glucose oxydase, une fructose oxydase, une saccarase et une galactose oxydase, le saccharose est décomposé par la saccarase en glucose, fructose et galactose, ces produits étant ensuite eux-mêmes décomposés par la cglucose oxydase, la fructose oxydase et la galactose oxydase.

Lors de l'étape d'apparition d'une différence de température 130, une différence de température apparaît, suite à la ou les réactions exothermiques, entre l'extrémité chaude et l'extrémité froide de chaque électrode 55, 60 comportant un élément 80. Plus précisément, la ou les réactions exothermiques entraînent un chauffage de l'extrémité chaude par rapport à l'extrémité froide.

Lors de l'étape d'apparition d'une différence de potentiel 140, une différence de potentiel électrique apparaît entre les deux deuxièmes extrémités 75 suite à l'apparition de la différence de température, par effet thermoélectrique.

En particulier, une tension électrique apparaît entre les extrémités chaude et froide de chaque électrode 55, 60 comportant un élément 80. Puisque les deux premières extrémités 70 sont électriquement connectées l'une à l'autre, une différence de potentiel est ainsi générée entre les deux deuxièmes extrémités 75.

La différence de potentiel est, par exemple, comprise entre 1 microvolt par degré Kelvin et 9 millivolts par degré Kelvin.

La différence de potentiel est transmise au convertisseur 30, par exemple via deux conducteurs électriques reliant chacun le convertisseur 30 à la deuxième extrémité 75 d'une électrode 55, 60.

Grâce à l'utilisation de l'élément 80, le générateur thermoélectrique 45 est susceptible de générer une différence de potentiel même lorsque le générateur thermoélectrique 45 est plongé dans un fluide F qui est homogène en température. Par exemple, lorsque le générateur thermoélectrique 45 est implanté dans le corps, le générateur thermoélectrique 45 est susceptible de délivrer une puissance électrique de l'ordre de plusieurs milliwatts bien que la température corporelle soit relativement homogène. De même, le générateur thermoélectrique 45 est susceptible de générer une puissance électrique importante lorsqu'il est plongé dans un flux rejeté d'une installation industrielle, bien que ce flux soit homogène en température.

Le générateur thermoélectrique 45 est donc susceptible de délivrer une puissance électrique importante même lorsque le générateur thermoélectrique 45 est dans un milieu dans lequel un générateur thermoélectrique de l'état de la technique ne produirait qu'une puissance électrique très faible ou nulle.

De plus, le générateur thermoélectrique 45 n'est pas limité à des milieux dans lesquels des oxydants et des réducteurs sont présents, mais est susceptible d'être utilisé dans un grand nombre de milieux tant que des espèces chimiques propres à réagir entre elles pour former une réaction exothermique sont disponibles, même si cette réaction n'est pas une réaction d'oxydoréduction.

Le générateur thermoélectrique 45 est donc adapté à un plus grand nombre de milieux que les générateurs thermoélectriques de l'état de la technique.

En outre, le générateur thermoélectrique 45 présente de petites dimensions et est donc plus adapté que d'autres types de générateurs électriques à des applications telles que l'alimentation électrique de dispositifs implantables.

Le deuxième boîtier 65 permet d'isoler thermiquement l'extrémité chaude de la majorité du fluide F, et permet donc d'atteindre aisément des températures plus élevées au niveau de l'extrémité chaude qu'en l'absence de deuxième boîtier 65. La puissance électrique générée est donc plus élevée qu'en l'absence de deuxième boîtier 65.

Les matériaux polymères sont particulièrement adaptés à la réalisation de deuxièmes boîtiers 65 thermiquement isolants. En outre, de nombreux polymères sont particulièrement bien tolérés par l'organisme humain ou animal.

Un deuxième boîtier 65 réalisé en un matériau poreux permet d'allier à la fois isolation thermique et simplicité de fonctionnement, puisqu'aucune pompe n'est nécessaire. En outre, la puissance électrique disponible en sortie du générateur thermoélectrique est plus élevée, puisqu'aucune puissance électrique n'est déroutée pour l'alimentation d'une pompe.

L'utilisation d'une pompe pour injecter le fluide F dans la chambre 85 permet d'augmenter le débit de fluide F qui entre en contact avec l'élément 80 par rapport à un cas où le déplacement du fluide F aurait lieu uniquement par convection. En outre, le débit de fluide F et donc la puissance électrique générée par le dispositif implantable 10 est susceptible d'être régulée en contrôlant la pompe.

Une pompe à membrane piézoélectrique consomme peu d'énergie et est très adaptée à des applications dans lesquels le volume de fluide F injecté dans la chambre 85 est faible, par exemple lorsque le générateur thermoélectrique 45 est implanté dans le corps.

Lorsque l'élément 80 est en forme de particules dispersées dans une matrice, ou un solide poreux formé de particules compactées, une grande quantité d'espèces chimiques C1, C2 est susceptible d'entrer en contact avec l'élément 80, et donc de participer à la réaction exothermique correspondante. Là encore, la puissance électrique ainsi générée est plus élevée.

Les bactéries et les levures étant capables de s'autoreproduire, les performances du générateur thermoélectrique 45 dans le temps sont moins susceptibles de diminuer qu'avec d'autres types d'éléments 80.

Les levures et les bactéries présentent également une bonne résistance à l'environnement dans lequel elles sont plongées, notamment si ces espèces sont naturellement présentes dans l'environnement dans lequel le dispositif implantable 10 est prévu pour être plongé, en particulier si ces espèces sont naturellement présentes dans le fluide F. C'est le cas par exemple des bactéries intestinales lorsque le dispositif implantable 10 est prévu pour être implanté dans l'intestin.

Les enzymes présentent une grande sélectivité dans les réactions catalysées et permettent donc un bon contrôle de ces réactions.

L'utilisation d'au moins deux réactions exothermiques successives dont l'une met en jeu les produits P de l'autre permet d'augmenter la température obtenue au niveau de l'extrémité chaude et donc d'augmenter la puissance électrique délivrée.

Le glucose est une molécule disponible dans de nombreux fluides F du corps, et donc particulièrement adaptée à être utilisée comme première espèce chimique C1 et/ou deuxième espèce chimique C2, d'autant plus que de nombreuses réactions exothermiques mettent en jeu le glucose et/ou les produits P résultant de sa décomposition.

Dans l'exemple ci-dessus, le générateur thermoélectrique 45 a été décrit dans un cas où le générateur thermoélectrique 45 comporte deux électrodes 55, 60. Il est à noter que des modes de réalisation dans lesquels le générateur thermoélectrique 45 comporte plus de deux électrodes 55, 60 sont envisageables.

Par exemple, le générateur thermoélectrique 45 comporte trois électrodes montées en série de telle sorte que la deuxième extrémité 75 de la deuxième électrode 60 est électriquement connectée à la deuxième extrémité 75 d'une troisième électrode. La troisième électrode est, en particulier, identique à la première électrode 55.

La différence de potentiel ainsi générée par le générateur thermoélectrique 45 est mesurée entre la deuxième extrémité 75 de la première électrode 55 et la première extrémité 70 de la troisième électrode. En particulier, une première différence de potentiel apparaît entre les deuxièmes extrémités 75 de la première électrode 55 et de la deuxième électrode 60, et une deuxième différence de potentiel apparaît entre la première extrémité et la deuxième extrémité de la troisième électrode.

Des modes de réalisation dans lesquels plus de trois électrodes montées en série sont présentes dans le générateur thermoélectrique 45 sont également envisageables.

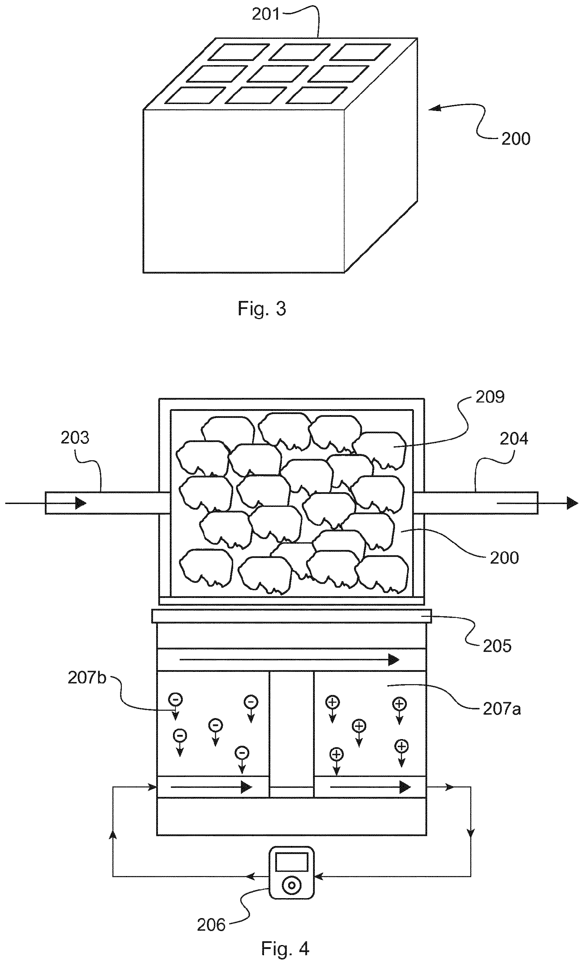

Si l'on se réfère à la Figure 3, on peut voir que l'on y a représenté une matrice 3D poreuse 200 qui peut être utilisée en tant que matériau poreux pour un générateur thermoélectrique selon la présente invention.

La matrice 3D poreuse 200 dans le mode de réalisation représenté est cubique, l'invention n'étant toutefois pas limitée à cet égard, la matrice 3D poreuse pouvant avoir également une forme de cylindre ou de pavé.

Les rectangles 201 représentés sur la matrice 3D poreuse 200 constituent les pores de la matrice 3D poreuse 200, La taille des pores de la matrice 3D poreuse peut varier de 50 $\mu$m jusqu'à 1mm.

La matrice 3D poreuse est par exemple fabriquée par impression 3D à partir d'une pâte visqueuse de poudre de particules métalliques et d'un liant visqueux, les particules de poudre de particules métalliques étant par exemple des particules de carbone, des particules de nanotubes de carbone, des particules de charbon actif, des particules de graphène, des particules d'aluminium.

Le liant visqueux est quant à lui l'un ou un mélange de plusieurs éléments parmi les composés suivants : dérivés de cellulose, alginate, agarose, alcool polyvinylique, polychlorure de vinyle.

Une face de la matrice 3D poreuse 200 est fermée par un isolant thermique, par exemple le pyralène, les autres faces de la matrice 3D poreuse étant fermées par un matériau thermoconducteur, par exemple l'aluminium.

Les bactéries ou micro-organismes pourront croître à l'intérieur des pores 201 de la matrice 3D poreuse 200,

La Figure 4 représente un générateur thermoélectrique 202 selon un mode de réalisation de la présente invention en utilisation avec une matrice 3D poreuse 200 selon la Figure 3,

Comme on peut le voir sur la Figure 4, la matrice 3D poreuse 200 comprend une entrée de fluide 203, une sortie de fluide 204, le fluide étant ici du glucose afin de créer entre l'entrée de fluide 203 et la sortie de fluide 204 un canal de glucose pour alimenter les bactéries ou microorganismes présents dans celle-ci, la porosité du matériau constituant la matrice 3D poreuse permettant également leur alimentation.

La matrice 3D poreuse 200 est une matrice en polymère de carbone à faible capacité thermique afin de rendre les pertes thermiques minimales dans le générateur thermoélectrique 202, pour optimiser la chaleur dégagée par les bactéries ou microorganismes 209 présents dans celle-ci. La matrice 3D poreuse est cubique dans le mode de réalisation non limitatif représenté, et cinq de ses faces sont recouvertes d'un isolant thermique, par exemple un polymère type parylène, et la dernière face 205 est recouverte d'un matériau thermoconducteur type aluminium par exemple.

Le générateur thermoélectrique 202 est quant à lui constitué d'une source de tension 206, de deux électrodes 207a et 207b, dont une extrémité est reliée à la source de tension, et dont l'autre extrémité est reliée à une plaque 208 de type matériau thermoconducteur type aluminium, pour créer, côté source de tension 206 une source froide et côté plaque 208 une source chaude. La plaque 208 en tant que source chaude est mise en contact ou à proximité de la face 205 de la matrice 3D poreuse 200 pour communiquer de la chaleur à celle-ci.

Des bactéries ou microorganismes 209 sont présents dans la matrice 3D poreuse 200. L'utilisation de la matrice poreuse 3D permet d'augmenter le nombre ou la densité des bactéries ou micro-organismes 209 par la création de biofilms bactériens ou de micro-organismes à la surface et à l'intérieur de la matrice 3D poreuse 200

La densité de bactéries ou micro-organismes 209 est en effet plus importante dans un biofilm que dans le cas où les bactéries ou micro-organismes sont en solution.

La porosité de la matrice 3D permet également avantageusement l'alimentation en eau et substrats des micro-organismes.

**Revendications**

1. Générateur thermoélectrique (45) destiné à être plongé dans un fluide (F) contenant au moins une première espèce chimique, le générateur thermoélectrique (45) comprenant au moins deux électrodes (55, 60), chaque électrode (55, 60) présentant une première extrémité (70) et une deuxième extrémité (75), les premières extrémités (70) étant électriquement connectées l'une à l'autre, le générateur (45) étant configuré pour générer une différence de potentiel électrique entre les deux deuxièmes extrémités (75) lorsqu'une différence de température est imposée entre chaque première extrémité (70) et la deuxième extrémité (75) de la même électrode (55, 60), la différence de température étant telle qu'une extrémité, appelée « extrémité chaude », parmi la première extrémité (70) et la deuxième extrémité (75) de chaque électrode (55, 60) présente une température strictement supérieure à la température de l'autre extrémité (70, 75), appelée « extrémité froide », parmi la première extrémité (70) et la deuxième extrémité (75) de la même électrode (55, 60),

   l'extrémité chaude d'au moins une électrode (55, 60) comportant au moins un élément (80) choisi parmi l'ensemble formé d'un micro-organisme et d'une enzyme, l'élément (80) étant propre à provoquer au moins une réaction exothermique mettant en jeu la première espèce chimique,
   le générateur thermoélectrique (45) étant **caractérisé en ce qu'**il comprend en outre un bloc en contact avec l'extrémité chaude d'au moins une électrode (55, 60), le bloc comprenant une matrice, la matrice étant notamment réalisée en un matériau polymère, l'élément (80) étant en forme de particules encapsulées dans la matrice.

2. Générateur thermoélectrique selon la revendication 1, comprenant un boîtier (65) délimitant une chambre (85) accueillant les deux extrémités chaudes, la chambre (85) accueillant en outre l'élément (80), le boîtier (65) étant configuré pour être traversé au moins par la première espèce chimique.

3. Générateur selon la revendication 2, dans lequel le boîtier (65) est réalisé en un matériau poreux.

4. Générateur selon la revendication 2 ou 3, comprenant une pompe propre à injecter un flux de fluide (F) depuis l'extérieur du boîtier (65) jusque dans la chambre (85).

5. Générateur selon l'une quelconque des revendications 2 à 4, dans lequel le boîtier (65) est réalisé en un matériau polymère.

6. Générateur selon l'une quelconque des revendications 3 et 4, dans lequel le matériaux poreux est constitué d'une matrice 3D poreuse.

7. Générateur selon la revendication 6, dans lequel la forme géométrique de la matrice est l'une parmi un cube, un cylindre, un pavé.

8. Générateur selon l'une des revendications 6 et 7, dans lequel 70% à 80% de la surface de la matrice 3D est couverte d'une couche thermiquement isolante.

9. Générateur selon l'une des revendications 6 à 8, dans lequel la matrice possède une entrée de fluide et une sortie de fluide.

10. Générateur selon l'une des revendications 6 à 9, dans lequel la matrice 3D comprend des micro-organismes qui forment un biofilm à l'intérieur de la matrice 3D.

11. Générateur thermoélectrique selon l'une des revendications 6 à 10, dans lequel la matrice poreuse est un bloc métal.

12. Générateur selon l'une quelconque des revendications 1 à 11, dans lequel l'élément (80) est propre à provoquer une première réaction exothermique et une deuxième réaction exothermique, la première réaction générant au moins un produit par réaction de la première espèce chimique, la deuxième réaction étant une réaction mettant en jeu le produit de la première réaction.

13. Générateur thermoélectrique selon l'une quelconque des revendications 1 à 12, dans lequel au moins un élément (80) est un micro-organisme, au moins une des propriétés suivantes étant vérifiée :

   - le micro-organisme comprend une levure, et
   - le micro-organisme comprend une bactérie.

14. Générateur selon l'une quelconque des revendications 1 à 13, dans lequel la première espèce chimique est choisie parmi l'ensemble formé de : l'urée, un alcool et un sucre.

15. Générateur selon la revendication 14, dans lequel au moins une espèce est le glucose.

16. Dispositif implantable (10) configuré pour être implanté dans le corps humain ou animal, comprenant un générateur thermoélectrique (45) selon l'une quelconque des revendications précédentes, dans lequel le fluide (F) est un fluide corporel du corps humain ou animal, la première espèce chimique étant notamment le glucose.

17. Dispositif implantable selon la revendication 16, dans lequel au moins un élément (80) comprend une enzyme, l'élément (80) comprenant une glucose oxydase et une catalase.

18. Dispositif implantable selon la revendication 16 ou 17, dans lequel le dispositif implantable (10) est un stimulateur cardiaque.

19. Procédé de génération d'une différence de potentiel électrique, comprenant des étapes de :

   - fourniture (100) d'un générateur thermoélectrique (45) comprenant au moins deux électrodes (55, 60), chaque électrode (55, 60) présentant une première extrémité (70) et une deuxième extrémité (75), les premières extrémités (70) étant électriquement connectées l'une à l'autre, le générateur (45) étant configuré pour générer une différence de potentiel électrique entre les deux deuxièmes extrémités (75) lorsqu'une différence de température est imposée entre chaque première extrémité (70) et la deuxième extrémité (75) de la même électrode (55, 60), la différence de température étant telle qu'une extrémité (70, 75), appelée « extrémité chaude », parmi la première extrémité (70) et la deuxième extrémité (75) de chaque électrode (55, 60) présente une température strictement supérieure à la température de l'autre extrémité (70, 75), appelée « extrémité froide » parmi la première extrémité (70) et la deuxième extrémité (75) de la même électrode (55, 60), l'extrémité chaude d'au moins une électrode (55, 60) comportant au moins un élément (80) choisi parmi l'ensemble formé d'un micro-organisme et d'une enzyme, le générateur thermoélectrique (45) comprenant en outre un bloc en contact avec l'extrémité chaude d'au moins une électrode (55, 60), le bloc comprenant une matrice, la matrice étant notamment réalisée en un matériau polymère, l'élément (80) étant en forme de particules encapsulées dans la matrice,
   - trempage (110) du générateur thermoélectrique (45) dans un fluide (F) comprenant au moins une première espèce chimique,
   - mise en œuvre (120), par l'élément (80), d'au moins une réaction exothermique mettant en jeu la première espèce chimique,
- apparition (130) d'une différence de température entre l'extrémité chaude et l'extrémité froide de l'électrode (55, 60) comportant l'élément (80), et
- apparition (140) d'une différence de potentiel électrique entre les deux deuxièmes extrémités (75).

**Patentansprüche**

**1.** - Thermoelektrischer Generator (45), der zum Eintauchen in ein Fluid (F) bestimmt ist, das mindestens eine erste chemische Spezies enthält, wobei der thermoelektrische Generator (45) mindestens zwei Elektroden (55, 60) umfasst, wobei jede Elektrode (55, 60) ein erstes Ende (70) und ein zweites Ende (75) aufweist, wobei die ersten Enden (70) miteinander elektrisch verbunden sind, wobei der Generator (45) ausgelegt ist, um eine elektrische Potentialdifferenz zwischen den zwei zweiten Enden (75) zu erzeugen, wenn eine Temperaturdifferenz zwischen jedem ersten Ende (70) und dem zweite Ende (75) derselben Elektrode (55, 60) angelegt ist, wobei die Temperaturdifferenz derart ist, dass ein Ende, bezeichnet als "warmes Ende", von dem ersten Ende (70) und dem zweiten Ende (75) jeder Elektrode (55, 60) eine Temperatur aufweist, die strikt höher als die Temperatur des anderen Endes (70, 75), bezeichnet als "kaltes Ende", von dem ersten Ende (70) und dem zweiten Ende (75) derselben Elektrode (55, 60) ist,

wobei das warme Ende mindestens einer Elektrode (55, 60) mindestens ein Element (80) aufweist, das aus einer Einheit ausgewählt ist, die von einem Mikroorganismus und von einem Enzym gebildet ist, wobei das Element (80) imstande ist, mindestens eine exotherme Reaktion hervorzurufen, bei der die erste chemische Spezies zum Einsatz kommt,
wobei der thermoelektrische Generator (45) **dadurch gekennzeichnet ist, dass** er ferner einen Block im Kontakt mit dem warmen Ende mindestens einer Elektrode (55, 60) umfasst, wobei der Block eine Matrix umfasst, wobei die Matrix insbesondere aus einem Polymermaterial hergestellt ist, wobei das Element (80) in Form von Partikeln vorliegt, die in der Matrix eingekapselt sind.

**2.** - Thermoelektrischer Generator nach Anspruch 1, umfassend ein Gehäuse (65), das eine Kammer (85) begrenzt, die die zwei warmen Enden aufnimmt, wobei die Kammer (85) ferner das Element (80) aufnimmt, wobei das Gehäuse (65) ausgelegt ist, um von mindestens der ersten chemischen Spezies durchquert zu werden.

**3.** - Generator nach Anspruch 2, wobei das Gehäuse (65) aus einem porösen Material hergestellt ist.

**4.** - Generator nach Anspruch 2 oder 3, umfassend eine Pumpe, die imstande ist einen Strom eines Fluids (F) von außerhalb des Gehäuses (65) bis in die Kammer (85) einzuleiten.

**5.** - Generator nach einem der Ansprüche 2 bis 4, wobei das Gehäuse (65) aus einem Polymermaterial hergestellt ist.

**6.** - Generator nach einem der Ansprüche 3 und 4, wobei das poröse Material aus einer porösen 3D-Matrix besteht.

**7.** - Generator nach Anspruch 6, wobei die geometrische Form der Matrix entweder ein Würfel, ein Zylinder oder ein Klotz ist.

**8.** - Generator nach einem der Ansprüche 6 und 7, wobei 70 % bis 80 % der Oberfläche der 3D-Matrix von einer thermisch isolierenden Schicht bedeckt ist.

**9.** - Generator nach einem der Ansprüche 6 bis 8, wobei die Matrix einen Fluideinlass und einen Fluidauslass besitzt.

**10.** - Generator nach einem der Ansprüche 6 bis 9, wobei die 3D-Matrix Mikroorganismen umfasst, die im Inneren der 3D-Matrix einen Biofilm bilden.

**11.** - Thermoelektrischer Generator nach einem der Ansprüche 6 bis 10, wobei die poröse Matrix ein Metallblock ist.

**12.** - Generator nach einem der Ansprüche 1 bis 11, wobei das Element (80) imstande ist, eine erste exotherme Reaktion und eine zweite exotherme Reaktion hervorzurufen, wobei die erste Reaktion mindestens ein Produkt durch Reaktion der ersten chemischen Spezies erzeugt, wobei die zweite Reaktion eine Reaktion ist, bei der das Produkt der ersten Reaktion zum Einsatz kommt.

**13.** - Thermoelektrischer Generator nach einem der Ansprüche 1 bis 12, wobei mindestens ein Element (80) ein Mikroorganismus ist, wobei mindestens eine der folgenden Eigenschaften überprüft wird:

- der Mikroorganismus umfasst eine Hefe, und
- der Mikroorganismus umfasst eine Bakterie.

**14.** - Generator nach einem der Ansprüche 1 bis 13, wobei die erste chemische Spezies aus einer Einheit ausgewählt ist, die von Harnstoff, einem Alkohol und einem Zucker gebildet ist.

**15.** - Generator nach Anspruch 14, wobei mindestens eine Spezies die Glucose ist.

**16.** - Implantierbare Vorrichtung (10), die ausgelegt ist, um in den Körper eines Menschen oder Tieres implantiert zu sein, umfassend einen thermoelektrischen Generator (45) nach einem der vorangehenden Ansprüche, wobei das Fluid (F) ein Körperfluid des Körpers des Menschen oder Tieres ist, wobei die erste chemische Spezies insbesondere die Glucose ist.

**17.** - Implantierbare Vorrichtung nach Anspruch 16, wobei mindestens ein Element (80) ein Enzym umfasst, wobei das Element (80) eine Glucose- Oxydase und eine Katalase umfasst.

**18.** - Implantierbare Vorrichtung nach Anspruch 16 oder 17, wobei die implantierbare Vorrichtung (10) ein Herzschrittmacher ist.

**19.** - Verfahren zum Erzeugen einer elektrischen Potentialdifferenz, umfassend die folgenden Schritte:

- Bereitstellen (100) eines thermoelektrischen Generators (45), der mindestens zwei Elektroden (55, 60) umfasst, wobei jede Elektrode (55, 60) ein erstes Ende (70) und ein zweites Ende (75) aufweist, wobei die ersten Enden (70) miteinander elektrisch verbunden sind, wobei der Generator (45) ausgelegt ist, um eine elektrische Potentialdifferenz zwischen den zwei zweiten Enden (75) zu erzeugen, wenn eine Temperaturdifferenz zwischen jedem ersten Ende (70) und dem zweite Ende (75) derselben Elektrode (55, 60) angelegt ist, wobei die Temperaturdifferenz derart ist, dass ein Ende (70, 75), bezeichnet als "warmes Ende", von dem ersten Ende (70) und dem zweiten Ende (75) jeder Elektrode (55, 60) eine Temperatur aufweist, die strikt höher als die Temperatur des anderen Endes (70, 75), bezeichnet als "kaltes Ende", von dem ersten Ende (70) und dem zweiten Ende (75) derselben Elektrode (55, 60) ist, wobei das warme Ende mindestens einer Elektrode (55, 60) mindestens ein Element (80) aufweist, das aus einer Einheit ausgewählt ist, die von einem Mikroorganismus und von einem Enzym gebildet ist, wobei der thermoelektrische Generator (45) ferner einen Block im Kontakt mit dem warmen Ende mindestens einer Elektrode (55, 60) umfasst, wobei der Block eine Matrix umfasst, wobei die Matrix insbesondere aus einem Polymermaterial hergestellt ist, wobei das Element (80) in Form von Partikeln vorliegt, die in der Matrix eingekapselt sind,
- Eintauchen (110) des thermoelektrischen Generators (45) in ein Fluid (F), das mindestens eine erste chemische Spezies umfasst,
- Durchführen (120), durch das Element (80), von mindestens einer exothermen Reaktion, bei der die erste chemische Spezies zum Einsatz kommt,
- Auftreten (130) einer Temperaturdifferenz zwischen dem warmen Ende und dem kalten Ende der Elektrode (55, 60) mit dem Element (80), und
- Auftreten (140) einer elektrischen Potentialdifferenz zwischen den zwei zweiten Enden (75).

**Claims**

**1.** - A thermoelectric generator (45) for immersion in a fluid (F) containing at least a first chemical species, the thermoelectric generator (45) comprising at least two electrodes (55, 60), each electrode (55, 60) having a first end (70) and a second end (75), the first ends (70) being electrically connected to each other, the generator (45) being configured to generate an electrical potential difference between the two second ends (75) when a temperature difference is imposed between each first end (70) and the second end (75) of the same electrode (55, 60), the temperature difference being such that one end, referred to as the "hot end", of the first end (70) and the second end (75) of each electrode (55, 60) has a temperature strictly higher than the temperature of the other end (70, 75), referred to as the "cold end", of the first end (70) and the second end (75) of the same electrode (55, 60),

the hot end of at least one electrode (55, 60) comprising at least one element (80) selected from the group consisting of a microorganism and an enzyme, the element (80) being suitable for causing at least one exothermic reaction involving the first chemical species,

the thermoelectric generator (45) being **characterised in that** it further comprises a block in contact with the hot end of at least one electrode (55, 60), the block comprising a matrix, the matrix being made in particular of a polymer material, the element (80) being in the form of particles encapsulated in the matrix.

2. - The thermoelectric generator according to claim 1, comprising a housing (65) defining a chamber (85) accommodating the two hot ends, the chamber (85) further accommodating the element (80), the housing (65) being configured to have at least the first chemical species pass through it.

3. - The generator according to claim 2, wherein the housing (65) is made of a porous material.

4. - The generator according to claim 2 or 3, comprising a pump suitable for injecting a flow of fluid (F) from outside the housing (65) into the chamber (85).

5. - The generator according to any one of claims 2 to 4, wherein the housing (65) is made of a polymeric material.

6. - The generator according to any one of claims 3 and 4, wherein the porous material consists of a porous 3D matrix.

7. - The generator according to claim 6, wherein the geometric shape of the matrix is one of a cube, a cylinder, a slab.

8. - The generator according to one of claims 6 and 7, wherein 70% to 80% of the surface of the 3D matrix is covered with a thermally insulating layer.

9. - The generator according to one of claims 6 to 8, wherein the matrix has a fluid inlet and a fluid outlet.

10. - The generator according to one of claims 6 to 9, wherein the 3D matrix comprises microorganisms that form a biofilm within the 3D matrix.

11. - The thermoelectric generator according to one of claims 6 to 10, wherein the porous matrix is a metal block.

12. - The generator according to any one of claims 1 to 11, wherein the element (80) is suitable for causing a first exothermic reaction and a second exothermic reaction, the first reaction generating at least one product by reaction of the first chemical species, the second reaction being a reaction involving the product of the first reaction.

13. - The thermoelectric generator according to any one of claims 1 to 12, wherein at least one element (80) is a microorganism, at least one of the following properties being verified:

  - the micro-organism comprises a yeast, and
  - the micro-organism comprises a bacterium.

14. - The generator according to any one of claims 1 to 13, wherein the first chemical species is selected from the group consisting of: urea, an alcohol and a sugar.

15. - The generator according to claim 14, wherein at least one species is glucose.

16. - An implantable device (10) configured to be implanted in the human or animal body, comprising a thermoelectric generator (45) according to any one of the preceding claims, wherein the fluid (F) is a body fluid of the human or animal body, the first chemical species being in particular glucose.

17. - The implantable device according to claim 16, wherein at least one member (80) comprises an enzyme, the member (80) comprising a glucose oxidase and a catalase.

18. - The implantable device according to claim 16 or 17, wherein the implantable device (10) is a pacemaker.

19. - A method of generating an electrical potential difference, comprising the steps of :

- providing (100) a thermoelectric generator (45) comprising at least two electrodes (55, 60), each electrode (55, 60) having a first end (70) and a second end (75), the first ends (70) being electrically connected to each other, the generator (45) being configured to generate an electrical potential difference between the two second ends (75) when a temperature difference is imposed between each first end (70) and the second end (75) of the same electrode (55, 60), the temperature difference being such that one end (70, 75), referred to as the "hot end", of the first end (70) and the second end (75) of each electrode (55, 60) has a temperature strictly higher than the temperature of the other end (70, 75), referred to as the "cold end", of the first end (70) and the second end (75) of the same electrode (55, 60), the hot end of at least one electrode (55, 60) comprising at least one element (80) selected from the group consisting of a micro-organism and an enzyme, the thermoelectric generator (45) further comprising a block in contact with the hot end of at least one electrode (55, 60), the block comprising a matrix, the matrix being made in particular of a polymeric material, the element (80) being in the form of particles encapsulated in the matrix,
- dipping (110) the thermoelectric generator (45) in a fluid (F) comprising at least a first chemical species,
- implementing (120), by the element (80), at least one exothermic reaction involving the first chemical species,
- the onset (130) of a temperature difference between the hot end and the cold end of the electrode (55, 60) comprising the element (80), and
- the onset (140) of an electrical potential difference between the two second ends (75).

Fig.1

EP 3 803 990 B1

Fig.2

Fig. 3

Fig. 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Thermoelectric enzyme sensor for measuring blood glucose. **MUEHLBAUER M J et al.** BIOSENSORS AND BIOELECTRONICS. ELSEVIER SCIENCE LTD, 01 Janvier 1990, vol. 5, 1-12